(19) **Europäisches Patentamt European Patent Office Office européen des brevets**

(11) **EP 2 559 708 B1**

(12) **EUROPEAN PATENT SPECIFICATION**

(45) Date of publication and mention of the grant of the patent:
**20.06.2018 Bulletin 2018/25**

(51) Int Cl.:
*C08B 37/08* (2006.01)    *A61K 8/73* (2006.01)
*A61Q 1/04* (2006.01)    *A61Q 1/14* (2006.01)
*A61Q 5/02* (2006.01)    *A61Q 17/04* (2006.01)
*A61Q 19/00* (2006.01)    *C08L 5/08* (2006.01)

(21) Application number: **11768891.1**

(22) Date of filing: **13.04.2011**

(86) International application number:
**PCT/JP2011/059191**

(87) International publication number:
**WO 2011/129370 (20.10.2011 Gazette 2011/42)**

(54) **PROCESS FOR PRODUCING ZINC HYALURONATE, PROCESS FOR PRODUCING COSMETIC CONTAINING ZINC HYALURONATE, ZINC HYALURONATE AND PROCESS FOR PRODUCING THE SAME**

VERFAHREN ZUR HERSTELLUNG VON ZINK-HYALURONAT, VERFAHREN ZUR HERSTELLUNG EINES KOSMETIKUMS ENTHALTEND ZINK-HYALURONAT, SOWIE ZINK-HYALURONAT UND DESSEN HERSTELLUNGSVERFAHREN

PROCÉDÉ POUR PRODUIRE DU HYALURONATE DE ZINC, PROCÉDÉ POUR PRODUIRE UN AGENT COSMÉTIQUE CONTENANT DU HYALURONATE DE ZINC, HYALURONATE DE ZINC ET PROCÉDÉ POUR LE PRODUIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2010   JP 2010129446
14.04.2010   JP 2010092883**

(43) Date of publication of application:
**20.02.2013   Bulletin 2013/08**

(73) Proprietors:
• **Kewpie Corporation
Tokyo 150-0002 (JP)**
• **Rohto Pharmaceutical Co., Ltd.
Osaka-shi
Osaka 544-8666 (JP)**

(72) Inventors:
• **OHNO, Atsushi
Fuchu-shi
Tokyo 183-0034 (JP)**
• **FUJIKAWA, Shunichi
Fuchu-shi
Tokyo 183-0034 (JP)**

• **YOSHIDA, Takushi
Fuchu-shi
Tokyo 183-0034 (JP)**
• **OE, Mariko
Fuchu-shi
Tokyo 183-0034 (JP)**
• **TAMURA, Masanori
Osaka-shi
Osaka 544-8666 (JP)**
• **YANAGISAWA, Miyuki
Osaka-shi
Osaka 544-8666 (JP)**
• **FUJIMOTO, Junichi
Osaka-shi
Osaka 544-8666 (JP)**

(74) Representative: **Winter, Brandl, Fürniss, Hübner, Röss, Kaiser, Polte - Partnerschaft mbB
Patent- und Rechtsanwaltskanzlei
Alois-Steinecker-Strasse 22
85354 Freising (DE)**

(56) References cited:
**EP-A1- 1 865 002        WO-A1-90/10020
WO-A1-2006/069578    WO-A1-2006/101030
WO-A1-2006/121210    CN-A- 1 760 214
US-A- 5 554 598**

**Description**

Technical Field

The present invention relates to a method for producing zinc hyaluronate, a method for producing a cosmetic preparation comprising the zinc hyaluronate, and a zinc hyaluronate and a method for producing the same.

Background Art

Zinc hyaluronate which is expected to be used for treatment of wound or burn wound, calcium hyaluronate, and copper hyaluronate, for example, are known as metal hyaluronate which is a complex of hyaluronic acid and a metal.
For example, patent No. JP2571312 B1 discloses a method for preparing a complex of deprotonated hyaluronic acid and a zinc ion or a cobalt ion.
CN 1760214 A discloses methods of preparation of powdery zinc hyaluronate by ionic exchange between sodium hyaluronate and 1-15% organic or inorganic of a zinc salt in a 40-100 vol% aqueous alcoholic solution. CN 1760214 A is silent on the average molecular weight of the sodium hyaluronate.

Summary of Invention

In the method disclosed in patent No. JP2571312 B1, an aqueous solution containing an equivalent of a zinc salt or a cobalt salt is added to an aqueous solution of sodium hyaluronate to obtain an associated product (complex) of the hyaluronic acid and the zinc ion or the cobalt ion by precipitation. In the method disclosed in patent No. JP2571312 B1, the associated product of the hyaluronic acid and the zinc ion or the cobalt ion is produced in such a state that the source sodium hyaluronate dissolves in the aqueous solution, and subsequently to be precipitated. Thus, it is difficult to precipitate the associated product completely, resulting in a low yield.
The present invention provides a method for producing a zinc hyaluronate, which can obtain the zinc hyaluronate in a simple method in a high yield, a method for producing a cosmetic preparation comprising the zinc hyaluronate, and a zinc hyaluronate and its salt thereof.
A method for producing a zinc hyaluronate according to one aspect of the invention comprises dispersing a hyaluronic acid and/or salt thereof in a water-containing medium comprising a zinc acetate at 15 to 60 °C at a pH of 3 to 9 for 10 minutes to 6 hours.
The term "zinc hyaluronate" used in the invention refers to a complex formed by interaction between a carboxyl ion ($COO^-$) originated from a carboxyl group constituting a hyaluronic acid and zinc ion.
In addition, the term "dispersing a hyaluronic acid and/or salt thereof in a water-containing medium" refers to making a part of the hyaluronic acid and/or salt thereof being present in the water-containing medium as a solid without dissolving, and preferably, making mostly of the hyaluronic acid and/or salt thereof being present in the water-containing medium as a solid without dissolving.
The method for producing the zinc hyaluronate may further comprise heating and drying a residue obtained by removing the water-containing medium.
In the above method for producing the zinc hyaluronate, the amount of the source hyaluronic acid and/or salt thereof in the water-containing medium is 5 to 500 mg/ml, the amount of the zinc acetate per volume of the water-containing medium is 5 to 500 mg/ml, the used amount of the zinc acetate for one part of the hyaluronic acid and/or salt thereof is 0.05 to 20 parts, and an average particle diameter, as measured by laser diffraction measurement, of the hyaluronic acid and/or salt thereof to be dispersed in the water-containing medium is 50 to 500 micrometers.
In the above method for producing the zinc hyaluronate, a water content in the water-containing medium is 10 to 55 vol %.
In the above method for producing the zinc hyaluronate, a medium constituting the water-containing medium is at least one selected from ethanol, methanol, and acetone.
In the above method for producing the zinc hyaluronate, the average molecular weight of the hyaluronic acid and/or salt thereof is 2,000 to 100,000.
A method for producing a cosmetic preparation according to another aspect of the invention comprises adding the zinc hyaluronate obtained by the above method for producing the zinc hyaluronate.
A zinc hyaluronate according to other aspect of the invention has a zinc content of 50 to 130 mg per 1 g of the zinc hyaluronate and a kinetic viscosity of a 1 % aqueous solution of the zinc hyaluronate is 0.3 to 5 $mm^2$/s.
The term "zinc hyaluronate" used in the invention refers to a complex formed by interaction between a carboxyl ion ($COO^-$) originated from a carboxyl group constituting a hyaluronic acid and a zinc ion ($Zn^{2+}$), and specifically, the complex can have a structure such that one zinc ion interacts with two hyaluronic acid (two carboxyl ion).
In the above zinc hyaluronate, the kinetic viscosity of a 1 % aqueous solution of the zinc hyaluronate may be 0.5 to 2 mm$^2$/s.

The above zinc hyaluronate may comprise 50 to 100 mg of the zinc per 1 g of the zinc hyaluronate.

The above zinc hyaluronate may be obtained by reacting a hyaluronic acid and/or salt thereof having an average molecular weight of 2,000 to 100,000 with a zinc compound.

A cosmetic preparation according to a further aspect of the invention comprises the above zinc hyaluronate.

Since the method for producing the zinc hyaluronate comprises dispersing a hyaluronic acid and/or salt thereof in a water-containing medium comprising a zinc acetate, the produced zinc hyaluronate is present in the water-containing medium as a solid without dissolving, thus the zinc hyaluronate can be easily purified and recovered. Accordingly, the zinc hyaluronate can be obtained in a simple method in a high yield.

Brief Description of Drawings

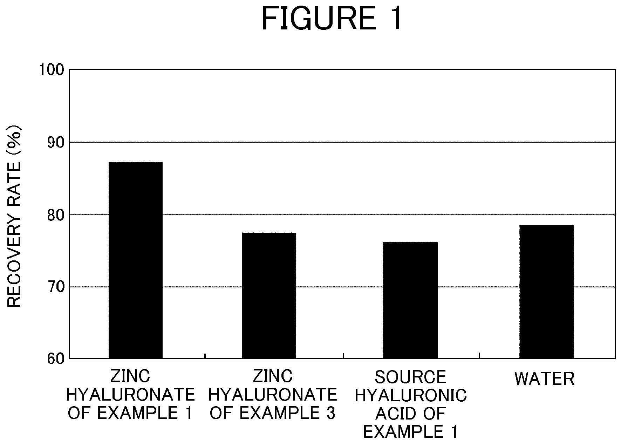

FIG. 1 shows a graph demonstrating the zinc hyaluronate of Example 1 can more effectively reduce water evaporation from the skin with a reduced barrier function, compared with the high-molecular-weight zinc hyaluronate of Example 3 and the source hyaluronic acid used in Example 1.

Description of Embodiments

A method for producing a zinc hyaluronate, a method for producing a cosmetic preparation comprising a zinc hyaluronate, and a zinc hyaluronate according to one embodiment of the invention are described in detail below. Furthermore, in the invention, "%" indicates "mass %" and "part" indicates "mass part".

1. Method for Producing Metal Hyaluronate (reference)

The method for producing a metal hyaluronate comprises dispersing a hyaluronic acid and/or salt thereof (hereinafter, occasionally referred as "source hyaluronic acid and/or salt thereof ") in a water-containing medium comprising a metallic compound comprising at least one selected from a metal of a group 2 element, a metal of a group 12 element, a metal of a group 13 element, and a metal of a transition element.

1.1. Dispersion Process

Since the method for producing the metal hyaluronate comprises dispersing the source hyaluronic acid and/or salt thereof in the water-containing medium comprising the metallic compound, the produced metal hyaluronate exists as a solid without dissolving in the aqueous medium, and thus the metal hyaluronate can be easily purified and collected. Accordingly, the metal hyaluronate can be obtained in a simple method in a high yield.

1.1.1. Source Hyaluronic Acid and/or Salt thereof

The source (raw material) used in the method for producing the metal hyaluronate according is hyaluronic acid and/or salt thereof having an average molecular weight of 2,000 to 100,000, more preferably 2,000 to 50,000, particularly preferably 4,000 to 20,000, most preferably 4,000 to 10,000. In general, as the average molecular weight of the source hyaluronic acid and/or salt thereof is smaller, the precipitation of the metal hyaluronate dissolving in an aqueous solution proceeds with difficulty even if alcohol or the like is added, and thus in this case, a method for obtaining metal hyaluronate by precipitation such as a method described in the specification of Patent No. JP2571312 for example results in a low yield. Thus, as the average molecular weight of the source hyaluronic acid and/or salt thereof is smaller, the advantage (that is, the metal hyaluronate can be obtained in a high yield) of the method for producing the metal hyaluronate is larger.

The term "hyaluronic acid" used in the invention refers to a polysaccharide including at least one constituent unit consisting of a glucuronic acid and a N-acetylglucosamine. Furthermore, "hyaluronic acid salt thereof (a salt of hyaluronic acid)" is not particularly limited, but preferably a salt being acceptable for food or a pharmaceutically acceptable salt, for example, a sodium salt, a potassium salt, an ammonium salt, and the like.

The source hyaluronic acid and/or salt thereof may be an extract obtained by extraction from living tissues such as animals (for example, cockscombs, umbilical cords, skin, joint fluid, etc.), or a culture obtained by culturing microorganisms, animal cells, or plant cells (for example, a fermentation method using Streptococcus microorganism, and the like), a chemical synthetic, or an enzymatic synthetic can be used.

The amount of the source hyaluronic acid and/or salt thereof in the water-containing medium is 5 to 500 mg/ml (preferably 20 to 200 mg/ml).

According to the invention, the average molecular weight of the source hyaluronic acid and/or salt thereof refers to a value measured by the following method.

Specifically, about 0.05 g of hyaluronic acid is weighed, and dissolved in a 0.2 mol/l sodium chloride solution to prepare a 100 mL of the resulting solution. A 0.2 mol/l sodium chloride solution is added to 8 mL, 12 mL, or 16 mL of the resulting solution to prepare each of the total amounts of 20 mL solutions. The each 20 mL solution and the resulting solution are used as sample solutions. The specific viscosity of each of the sample solutions and a 0.2 mol/l sodium chloride solution are measured at 30.0+-0.1 °C by the viscosity measurement method (first method (capillary viscosity measurement method)) of the general tests of Japanese Pharmacopoeia (15th edition) (expression 1), and the reduced viscosity at each concentration is calculated (expression 2). The reduced viscosity (vertical axis) and the dry matter concentration (g/100 mL) (horizontal axis) are plotted on a graph, and the limiting viscosity is calculated from the intersection point of a straight line that connects each point and the vertical axis. The limiting viscosity thus calculated is substituted into the Laurent's formula (expression 3) to calculate the average molecular weight (T. C. Laurent, M. Ryan, and A. Pietruszkiewicz, B. B. A., 42, 476-485 (1960)).

$$\texttt{Specific viscosity = [(falling time of sample solution) / (falling time of 0.2 mol/l sodium chloride solution)]-1 (expression 1)}$$

$$\texttt{Reduced viscosity (dL/g) = specific viscosity / (dry matter concentration (g/100 mL)) (expression 2)}$$

$$\texttt{Limiting viscosity (dL/g) = } 3.6 \times 10^{-4} \texttt{M}^{0.78} \texttt{ (expression 3)}$$

M: average molecular weight

1.1.2. Metallic Compound

The Metallic Compound used in the above dispersing process may be for example, at least one selected from a metal of a group 2 element, a metal of a group 11 element, a metal of a group 12 element, and a metal of a group 13 element.

As the metallic compound used in the above dispersing process, the metal of the group 2 element may be for example, magnesium, calcium, and barium, the metal of the group 12 element may be for example, zinc, the metal of the group 13 element may be for example, aluminum. Additionally, the metal of the transition element is the metal of the groups 3 to 11, and the metal of the group 3 element may be for example, a rare earth element selected from scandium, yttrium, and a lanthanoid element, and an actinoid element, the metal of the group 4 element may be for example, titanium, zirconium and hafnium, the metal of the group 5 element may be for example, vanadium, niobium, and tantalum, the metal of a group 6 element may be for example, chromium, molybdenum, and tungsten, the metal of a group 7 element may be for example, manganese, technetium, and rhenium, the metal of a group 8 element may be for example, iron, ruthenium, and osmium, the metal of a group 9 element may be for example, cobalt, rhodium, and iridium, the metal of a group 10 element may be for example, nickel, palladium, and platinum, and the metal of a group 11 element may be for example, copper, silver, and gold.

The metallic compound can be used alone or in combination of two or more. In particular, for example, at least one selected from a zinc compound, a magnesium compound, a calcium compound, a cobalt compound, a silver compound, and a copper compound is preferred as the metallic compound, from the viewpoint of versatility and availability of the obtained metal hyaluronate.

The zinc compound includes for example, an inorganic zinc such as zinc sulfonate, zinc chloride, zinc carbonate, and zinc oxide, and an organic zinc such as zinc acetate, zinc formate, and zinc laurate.

The magnesium compound includes for example, magnesium acetate, magnesium carbonate, magnesium chloride, magnesium sulfonate, magnesium lactate, magnesium hydroxide, and magnesium oxide.

The calcium compound includes for example, calcium carbonate, calcium chloride, calcium lactate, calcium gluconate, and calcium hydroxide.

The barium compound includes for example, barium hydroxide, barium chloride, and barium sulfide.

The copper compound includes for example, copper acetate, copper gluconate, and copper sulfate.

The silver compound includes for example, silver acetate, silver nitrate, silver sulfate, silver carbonate, and silver oxide.

The gold compound includes for example, chlorauric acid, gold cyanide, and gold potassium cyanide.

The aluminum compound includes for example, aluminum acetate, aluminum carbonate, aluminum sulfate, aluminum lactate, and aluminum chloride.

The cobalt compound includes for example, cobalt acetate.

The metallic compound is not particularly limited, but is preferably a metallic salt of an organic acid or a metallic salt of an inorganic acid, and an organic acid ion constituting the metallic salt of the organic acid or an inorganic acid ion constituting the metallic salt of the inorganic acid is more preferably a weak acid ion (for example, acetate ion, formate ion, carbonate ion). When the organic acid ion constituting the metallic salt of the organic acid or the inorganic acid ion constituting the metallic salt of the inorganic acid is a weak acid ion, the percentage of the organic acid or the inorganic acid without dissociation in the water-containing medium is larger than strong acids such as hydrochloric acid or sulfuric acid for example, and thus the metallic ion dissociating from the obtained metal hyaluronate is reduced and the yield of the metal hyaluronate can be increased.

For example, when the metallic compound is a zinc salt of an organic acid, the metallic compound is preferably a zinc salt of a weak organic acid, such as zinc acetate, zinc formate, while when the metallic compound is a zinc salt of an inorganic acid, the metallic compound is preferably a zinc salt of a weak inorganic acid such as zinc carbonate.

The metallic compound having a property to dissolve in water-containing medium is preferred as the metallic compound, in terms of being excellent in reactivity with the source hyaluronic acid and/or salt thereof.

The amount of the metallic compound per the volume of the water-containing medium is 5 to 500 mg/ml (preferably 20 to 200 mg/ml) . In addition, the used amount of the metallic compound for one part of the source hyaluronic acid and/or salt thereof is 0.05 to 20 parts (preferably 0.2 to 5 part).

1.1.3. Aqueous Medium

In the method for producing the metal hyaluronate, the water content in the water-containing medium is 20 to 55 vol %, preferably 20 to 40 vol %, in terms of enhancing the yield of the metal hyaluronate. In this case, if the water content in the water-containing medium is less than 5 vol %, the yield of the metal hyaluronate may be reduced, in contrast, if the water content in the water-containing medium is more than 55 vol %, the metal hyaluronate may have difficulty in precipitating, and as a result, the yield of the metal hyaluronate may be reduced. For example, when the metal hyaluronate is a zinc hyaluronate, the water content in the water-containing medium is preferably 20 to 55 vol %.

Additionally, the medium used in the water-containing medium is at least one selected from ethanol, methanol, and acetone.

The reaction temperature of the dispersion process is 15 to 60 °C, preferably 20 to 45 °C. If the reaction temperature is less than 15 °C, the metal content in the metal hyaluronate may be reduced. The reaction time of the dispersion process is 10 minutes to 6 hours.

The pH of the water-containing medium containing the metallic compound for dispersing the source hyaluronic acid and/or salt thereof is 3 to 9, preferably 4 to 8, more preferably 5 to 7. In this case, if the pH of the water-containing medium is less than 3, the metal content in the metal hyaluronate may be substantially reduced and the molecular weight of the metal hyaluronate may be substantially reduced, in contrast, if the pH of the water-containing medium is more than 9, the metal content in the metal hyaluronate may be substantially reduced.

1.2. Heating and Drying

The method for producing the metal hyaluronate may further comprise heating and drying a residue obtained by removing the water-containing medium after the dispersing process.

The reaction liquid obtained by the dispersion process keeps still standing to precipitate, and the water-containing medium is removed by decantation to obtain the residue. Next, a water-containing medium is added to this residue likewise, and if necessary, a combination of the precipitation by still-standing and the subsequent decantation may be performed once or more.

In addition, the temperature in the heating and drying process is not particularly limited, but usually 60 to 95 °C. Furthermore, the heating and drying may be performed under a reduced pressure.

1.3. Target Compound (Metal Hyaluronate)

The metal hyaluronate may be obtained by the method for producing the metal hyaluronate.

The average molecular weight of the metal hyaluronate is 2,000 to 100,000, preferably 2,000 to 50,000, more preferably 4,000 to 20,000, the most preferably 4,000 to 10,000.

The metal hyaluronate may be at least one selected from a metal of a group 2 element, a metal of a group 12 element, a metal of a group 13 element, and metal of transition element, for example, the metal hyaluronate may be at least one selected from a metal of a group 2 element, a metal of a group 9 element, a metal of a group 12 element, and a metal of a group 13 element, and in particular, the metal hyaluronate may contain at least one metal selected from zinc, magnesium, calcium, cobalt, silver, and copper. Specifically, the metal hyaluronate is a complex formed by an interaction of a carboxyl ion (COO-) originating from a carboxyl group constituting hyaluronic acid and a metal ion ($M^{a+}$, wherein the M is a metal element, and a is a valency represented by an integer of 1 to 3.)

2. Method for Producing Cosmetic Preparation Comprising Metal Hyaluronate (reference)

A method for producing a cosmetic preparation comprising a metal hyaluronate comprises adding the metal hyaluronate obtained by the method for producing the metal hyaluronate.

The cosmetic preparation comprising the metal hyaluronate, which is obtained by the method generally contains 0.001 to 5 % of the metal hyaluronate. Aspects of the cosmetic preparation includes for example, lotion (for example, whitening lotion), cream (for example, vanishing cream and cold cream), milky lotion, essence, pack (for example, jellied pack, wipe-off-type pasty pack, and rinse-type powdery pack), facial wash, a cleansing preparation, a cleansing product, foundation, rouge, lip balm, lip gloss, lip liner, cheek rouge, after-shave lotion, after-sun lotion, deodorant lotion, body lotion (including hand care lotion and foot care lotion), body oil, soap, a bath additive, shampoo, conditioner, hair pack, hair tonic, pilatory, perm solution, and coloring solution.

The cosmetic preparation comprising the metal hyaluronate obtained by the method may further comprise the following ingredients. The ingredients includes for example, a cationized polysaccharide (e.g., cationized hyaluronic acid, cationized hydroxyethyl cellulose, cationized guar gum, cationized starch, cationized locust bean gum, cationized dextran, cationized chitosan, and cationized honey), an anionic surfactant (e.g., alkylbenzenesulfonate, polyoxyalkylenealkylether sulfate salt, alkyl sulfate ester salt, olefin sulfonate, fatty acid salt, and dialkylsulfosuccinate salt), a nonionic surfactant (e.g., polyoxyethylene fatty acid ester and polyoxyethylene hydrogenated castor oil derivative), a cationic surfactant (e.g., alkyltrimethylammonium salt, dialkyldimethylammonium salt, alkylpyridinium salt, and stearyltrimethylammonium chloride), an amphoteric surfactant (e.g., alkyl betaine, alkylamide propyl betaine, imidazolinium betaine, egg-yolk lecithin, and soybean lecithin), oils (e.g., silicone, silicone derivatives, liquid paraffin, squalane, yellow bees wax, carnauba wax, olive oil, avocado oil, camellia oil, jojoba oil, and horse oil), a moisturizer (e.g., sodium hyaluronate, hydrolyzed hyaluronic acid, hydrophobized hyaluronic acid, acetylated hyaluronic acid, dimethylsilanol hyaluronate, ceramide, lauroyl glutamate diphytosteryloctyldodecyl, phytoglycogen, hydrolyzed eggshell membrane, trehalose, glycerol, atelocollagen, sorbitol, maltitol, and 1,3-butylene glycol), a higher fatty acid (e.g., lauric acid, behenic acid, palmitic acid, stearic acid, isostearic acid, and oleic acid), a higher alcohol (e.g., cetyl alcohol, stearyl alcohol, behenyl alcohol, isostearyl alcohol, and batyl alcohol), a polyhydric alcohol (e.g., glycerol, diglycerol, 1,3-propanediol, propylene glycol, polyethylene glycol, and pentylene glycol), a thickener (e.g., cellulose ether, carboxyvinyl polymer, xanthan gum, and dextrin palmitate), an amphoteric polymer resin compound (e.g., betaine dialkylaminoalkyl acrylate copolymer), a cationic polymer resin compound (e.g., cationized vinylpyrrolidone/dimethylaminoethyl methacrylate copolymer and polydimethyldiallylammonium halide cationic polymer), a preservative (e.g., methylparaben, ethylparaben, butylparaben, propylparaben, and phenoxyethanol, isopropylmethylphenol), an antioxidant (e.g., tocopherol, ascorbic acid, and BHT), a sequestering agent (e.g., edetate and etidronate), a UV absorber (e.g., benzophenone derivative, p-aminobenzoic acid derivative, and methoxycinnamic acid derivative), a UV reflection agent (e.g., titanium oxide and zinc oxide), a protein hydrolyzate (e.g., keratin peptide, collagen peptide, soybean peptide, wheat peptide, milk peptide, silk peptide, and egg white peptide), an amino acid (e.g., arginine, glutamic acid, glycine, alanine, hydroxyproline, cysteine, serine, and L-theanine), a natural extract (Sophorae radix extract, chamomile extract, seaweed extract, eucalyptus extract, royal jelly extract, Rosmarinus officinalis L. extract, and beech extract), other functional components (coenzyme Q10, polyquaternium-51, elastin, platinum nanocolloid, retinol palmitate, panthenol, allantoin, sodium lysine dilauroyl glutamate, magnesium ascorbyl phosphate, L-ascorbic acid 2-glucoside, ellagic acid, kojic acid, linoleic acid, tranexamic acid, hydroquinone, and hydroquinone derivative), a phospholipid polymer, essence, and a dye.

3. Zinc Hyaluronate and Method for Producing the same

3.1. Zinc Hyaluronate

A zinc hyaluronate according to this embodiment has a zinc content of 50 to 130 mg per 1 g of the zinc hyaluronate and a kinetic viscosity of a 1 % aqueous solution of the zinc hyaluronate is 0.3 to 5 mm$^2$/s.

A kinetic viscosity of the metal hyaluronate can be measured using an Ubbelohde viscometer (manufactured by Sibata Scientific Technology Ltd.). In this case, an Ubbelohde viscometer having such a coefficient that the falling time is 200 to 1,000 seconds is selected. The kinetic viscosity is measured in an incubator at 30 °C while maintaining a constant temperature. A kinetic viscosity of a 1 % aqueous solution of the zinc hyaluronate indicates the size of the molecular weight. In general, as the molecular weight of the zinc hyaluronate is larger, the kinetic viscosity of the 1 % aqueous solution is larger.

The kinetic viscosity (unit: $mm^2/s$) can be calculated by multiplying the falling time (sec) of the aqueous solution measured using the Ubbelohde viscometer by the coefficient of the Ubbelohde viscometer.

The kinetic viscosity of the 1 % aqueous solution of the zinc hyaluronate according to this embodiment is preferably 0.5 to 2 $mm^2/s$, and more preferably 1 to 1.5 $mm^2/s$. If the kinetic viscosity of the 1 % aqueous solution of the zinc hyaluronate according to this embodiment is more than 5 $mm^2/s$, the zinc hyaluronate according to this embodiment may have difficulty in penetrating into the corneum when the zinc hyaluronate is applied to the skin.

The zinc content per 1 g of the zinc hyaluronate according to this embodiment is preferably 55 to 100 mg, more preferably, 60 to 80 mg. The zinc content per 1 g of zinc hyaluronate according to this embodiment is a value measured by the method described later in Examples (chelate titration). The chelate titration is described in zinc sulfate hydrate (C-4675 to 4678) of respective articles of medicine in Japanese Pharmacopoeia (15th edition), for example.

The viscosity average molecular weight of the zinc hyaluronate according to this embodiment is 2,000 to 100,000, preferably 2,000 to 50,000, more preferably 4,000 to 10,000.

Further, the zinc hyaluronate according to this embodiment can be obtained by reacting a source hyaluronic acid and/or salt thereof having an average molecular weight of 2,000 to 100,000 with a zinc compound. The reaction is described in details later in the column "3.2. Method for Producing Zinc Hyaluronate".

It is reported that zinc hyaluronate have a beneficial effect on healing wound (for example, Patent No. JP2571312). The zinc hyaluronate according to this embodiment have a beneficial effect on healing wound and improving the skin texture, for example. 3.2. Method for Producing Zinc Hyaluronate

The method for producing a zinc hyaluronate according to the embodiment comprises dispersing a source hyaluronic acid and/or salt thereof having an average molecular weight 2,000 to 100,000 (preferably 2, 000 to 50, 000, more preferably 4, 000 to 20, 000, still more 4,000 to 10,000) in a water-containing medium comprising a zinc compound.

The conditions of the method for producing the zinc hyaluronate according to this embodiment are the same as the conditions of the method for producing the metal hyaluronate according to the above-described embodiment. Namely, the amount of the source hyaluronic acid and/or salt thereof, the amount of the water-containing medium, and the amount of the zinc compound, the reaction time, and the reaction temperature are the same as the amount of the source, the amount of the water-containing medium, and the amount of the metallic compound, the reaction time, and the reaction temperature in the method for the above-described method for producing the metal hyaluronate.

In the method for producing the zinc hyaluronate according to this embodiment, the average particle diameter of powder consisting of the source hyaluronic acid and/or salt thereof is 50 to 500 micrometers, preferably 100 to 400 micrometers. In this case, if the average particle diameter of the powder consisting of the source hyaluronic acid and/or salt thereof is less than 50 micrometers, the yield of the zinc hyaluronate may be reduced, in contrast, if the average particle diameter of the powder consisting of the source hyaluronic acid and/or salt thereof is more than 500 micrometers, the substitution of the source hyaluronic acid and/or salt thereof to the zinc hyaluronate may not proceed, and as a result, the zinc content may be reduced.

According to the invention, a measuring method of the average particle diameter of the powder consisting of the source hyaluronic acid and/or salt thereof may include for example, laser diffraction measurement or dynamic light scattering measurement, which is generally known. A method for adjusting the average particle diameter of the powder consisting of the source hyaluronic acid and/or salt thereof is not particularly limited. For example, a solution comprising hyaluronic acid and/or salt thereof is treated with a drying process such as spray drying, freeze drying, and then if necessary, being treated with crushing process.

Further, in the method for producing the zinc hyaluronate according to this embodiment, the water content in the water-containing medium is 20 to 55 vol %, from the viewpoint of obtaining the zinc hyaluronate having a high zinc content in a high yield.

4. Cosmetic Preparation

A cosmetic preparation according to one embodiment of the invention comprises the zinc hyaluronate according to the above embodiment.

Aspects of the cosmetic preparation include for example, those exemplified in the above-described "2. Method for Producing Cosmetic Preparation Comprising Metal Hyaluronate". The cosmetic preparation according to this embodiment may also include the ingredients exemplified in the above-described "2. Method for Producing Cosmetic Preparation Comprising Metal Hyaluronate", in addition to the zinc hyaluronate.

Since the cosmetic preparation according to this embodiment comprises the zinc hyaluronate, the cosmetic preparation can provide moisture to the skin, to improve the skin texture.

5. Examples

The invention is further described below by way of the following examples. Note that the invention is not limited to the examples. 5.1. Test Method

5.1.1. Average Molecular Weight

In the following examples, the average molecular weight of the source hyaluronic acid was measured by the method described in the above embodiment.

5.1.2. Yield of Metal Hyaluronate

In the following examples, the yield of the metal hyaluronate was a value obtained from the following formula.

```
Yield of Metal Hyaluronate (%) = Mass of Metal Hyaluronate / Mass of Source Hyaluronic Acid x 100 (%)
```

5.1.3. Zinc Content (mg) per 1 g of Zinc Hyaluronate

In the following examples, the zinc content (mg) per 1 g of zinc hyaluronate was measured based on chelate titration. The measuring procedure is described as follows.
0.3 g of zinc hyaluronate is precisely weighed (sample amount), and pure water was added to obtain an exact 100 mL of solution. 25 mL of the solution is poured into a 300 mL beaker, and then 100 mL of pure water and 2mL of ammoniacal ammonium chloride buffer solution are added. Next, 0.04 g of Eriochrome Black T•sodium chloride indicator is added and dissolved in the solution, and then titration is performed using 0.01 mol/L of EDTA solution to confirm change from red purple to powder blue visually. Reduction in dried weight of zinc hyaluronate which is a measuring object is measured in accordance with the method described in item (2) of sodium hyaluronate in manufacturing material specification for quasi drug (page 589 to 592) (reduced pressure, phosphorus pentaoxide, 60 °C, 4 hours).

```
Zinc Content per 1 g of Zinc Hyaluronate (mg/g) = {0.654 x Titer

of EDTA (mL) x 1.004 x (100/25)} /{Sample Amount (g) x (1- Reduction

in Dried Weight of Zinc Hyaluronate / 100))
```

5.1.4. Average Particle Diameter

In the following examples, the average particle diameter of the source hyaluronic acid was measured by laser diffraction described in the above embodiment. Specifically, the average particle diameter of the source hyaluronic acid was measured by using an instrument "SALD-2000J" (manufactured by Shimadzu Corporation) and ethanol was used as a dispersion medium.

5.1.5. Kinetic Viscosity

The kinetic viscosity of 1 % of zinc hyaluronate aqueous solution was measured in accordance with the equipment and the method described in the above embodiment.

5.2. Example 1

3 g of zinc acetate dihydrate $((CH_3CO_2)_2Zn•2H_2O)$ and 50 mL of pure water were charged into a 200 mL beaker and the zinc acetate dihydrate was dissolved in the water. After the zinc acetate dihydrate was dissolved in the water completely, 100 mL of ethanol was added to the solution to obtain a water-containing medium, and the water-containing medium (pH 6.3) was heated to a liquid temperature of 40 °C while stirring. After the liquid temperature reached to 40 °C, 5 g of source hyaluronic acid (average molecular weight: approximately 8,000, average particle diameter: approximately 230 micrometers, manufactured by Kewpie Corporation) was poured into a tank while stirring, the reaction liquid was stirred at 40 °C for 60 minutes to disperse the powder of the source hyaluronic acid in the reaction liquid. Furthermore, the pH of the each water-containing medium used for dispersing the hyaluronic acid in Comparative Examples 1 to 3, Examples 2 and 3, and Test Examples 1 to 3 described later is about the same as the pH of the water-containing medium of Example 1.

The reaction liquid was kept stationary for 15 minutes after the completion of the reaction, and then the supernatant water-containing ethanol containing zinc acetate was removed by decantation to obtain a precipitate. 110 mL of 90 %(v/v) ethanol was added to the obtained precipitate, and the mixture was stirred for 10 minutes in order to remove unreacted zinc and was kept stationary for 15 minutes, and then the supernatant water-containing ethanol containing 90 % of water was removed by decantation. This manipulation was repeated five times. The obtained precipitate (residue) was dried in vacuo at 60 °C to obtain zinc hyaluronate.

The yield of the zinc hyaluronate obtained in Example 1 was 108 %, and the zinc content per 1 g of zinc hyaluronate was 72.2 mg. In addition, the kinetic viscosity of the 1 % aqueous solution of the obtained zinc hyaluronate was 1.2 mm$^2$/s.

The zinc content per 1 g of zinc hyaluronate (theoretical value) obtained in Example 1 was 79.6 mg and the substitution rate for the theoretical value was 91 %. Each theoretical value of the metal content per 1 g of metal hyaluronate was calculated in accordance with the following method.

If the valency number of the metal ion is M (wherein M represents an integer of 1 or more), the theoretical value of the metal content per 1 g of metal hyaluronate was a value calculated on the basis that one constitutional unit of hyaluronic acid in the metal hyaluronate contains 1/M metal ion. For example, if the valency number of the metal ion is 1, the theoretical value of the metal content per 1 g of metal hyaluronate was a value calculated on the basis that one constitutional unit of hyaluronic acid in the metal hyaluronate contains one metal ion.

```
Metal Content (Theoretical Value) per 1 g of Metal Hyaluronate

(mg/g) = (Molecular Weight of Attached Metal / Valency Number of

Attached Metal) / (Molecular Weight of Sodium Hyaluronate - Molecular Weight of Sodium + (Molecular Weight of Attached Metal / Valency Number of Attached metal))
```

### 5.3. Comparative Example 1

5 g of a source hyaluronic acid (average molecular weight: approximately 8,000, average particle diameter: approximately 230 micrometers, manufactured by Kewpie Corporation), 3 g of zinc acetate dihyrate, 120 mL of pure water, 30 mL of ethanol were put into a one-liter beaker. After the hyaluronic acid and zinc acetate dihyrate were completely dissolved, the mixture was heated while stirring until the temperature of the liquid reached to 40 °C, and the reaction was performed for 60 minutes at a temperature of 40 °C.

After the completion of the reaction, 500 mL of ethanol was added slowly while stirring to suspend the mixed liquid and the suspension was collected by filtration. Next, the collected product was washed with 500 mL of 80 % (v/v) ethanol three times, and the obtained precipitation (residue) was dried in vacuo at 60 °C to obtain a zinc hyaluronate.

The yield of the zinc hyaluronate obtained in Comparative Example 1 was 79 %, the zinc content per 1 g of zinc hyaluronate was 78.4 mg. In addition, the kinetic viscosity of the 1 % aqueous solution of the obtained zinc hyaluronate was 1.2 mm$^2$/s.

### 5.4. Comparative Example 2

Comparative Example 2 was performed with reference to the method described in Example 1 of JP 2009-278259.

At first, the hyaluronic acid was reacted with zinc acetate dihyrate in the similar method to Comparative Example 1, and then the pH of the solution was adjusted to 1.0 with hydrochloric acid. Next, 200 mL of ethanol was added slowly while stirring to separate a suspension phase and a supernatant phase. Then, the pH was adjusted to 5.0 with sodium hydroxide, and then the resulting precipitate was collected by filtration, and washed with 100 mL of 80 % (v/v) ethanol three times. The obtained precipitation (residue) was dried in vacuo at 60 °C to obtain a zinc hyaluronate.

The yield of the zinc hyaluronate obtained in Comparative Example 2 was 86 %, the zinc content per 1 g of zinc hyaluronate was 8.7 mg. In addition, the kinetic viscosity of the 1 % aqueous solution of the obtained zinc hyaluronate

was 1.1 mm$^2$/s.

5.5. Example 2 (reference example)

Zinc hyaluronate was manufactured by the similar method to Example 1 except hyaluronic acid (molecular weight: approximately 1,800,000, average particle diameter: approximately 450 micrometers, manufactured by Kewpie Corporation) was used as a source in Example 1.

The yield of the zinc hyaluronate obtained in Example 2 was 109 %, the zinc content per 1 g of zinc hyaluronate was 50.8 mg. In addition, the kinetic viscosity of the 1 % aqueous solution of the obtained zinc hyaluronate was 180 mm$^2$/s.

5.6. Comparative Example 3

5 g of a source hyaluronic acid (average molecular weight: approximately 1,800,000, average particle diameter: approximately 500 micrometers, manufactured by Kewpie Corporation), 3 g of zinc acetate dihyrate, 120 mL of pure water, 30 mL of ethanol were put into a one-liter beaker. After the hyaluronic acid and the zinc acetate dihyrate were completely dissolved, the mixture was heated while stirring until the temperature of the liquid reached to 40 °C, the reaction was performed for 60 minutes at a temperature of 40 °C.

After the completion of the reaction, 500 mL of ethanol was added slowly while stirring to precipitate zinc hyaluronate, and the supernatant liquid was removed by decantation to collect a precipitate. Next, the precipitate was washed with 500 mL of 80 % (v/v) ethanol three times, and the obtained precipitation (residue) was dried in vacuo at 60 °C to obtain the zinc hyaluronate.

The yield of the zinc hyaluronate obtained in Comparative Example 3 was 88 %, the zinc content per 1 g of zinc hyaluronate was 62.4 mg. In addition, the kinetic viscosity of the 1 % aqueous solution of the obtained zinc hyaluronate was 175 mm$^2$/s.

5.7. Test Example 1

Each zinc hyaluronate (No. 1 to 8) was manufactured by the similar method to Example 1 except hyaluronic acid (molecular weight: approximately 8,000, average particle diameter: approximately 200 micrometers, manufactured by Kewpie Corporation) was used as a source (raw material) in Example 1 and the water amount and the ethanol amount in the water-containing medium (water-containing ethanol) before adding the hyaluronic acid were changed as shown in Table 1. Test No. 8 is a reference example.

[Table 1]

| No. | Water Content in Water-containing Medium (mL) | Ethanol Content in Water-containing Medium (mL) | Water Proportion in Water-containing Medium (%) | Yield of Zinc Hyaluronate (%) | Zinc Content per 1 g of Zinc Hyaluronate (mg) |
|---|---|---|---|---|---|
| 1 | 80 | 70 | 53.3 | 94 | 73.4 |
| 2 | 70 | 80 | 46.7 | 101 | 70.3 |
| 3 | 60 | 90 | 40 | 104 | 69.4 |
| 4 | 50 | 100 | 33.3 | 106 | 66.7 |
| 5 | 40 | 110 | 26.7 | 105 | 62.8 |
| 6 | 30 | 120 | 20 | 103 | 55.4 |
| 7 | 20 | 130 | 13.3 | 102 | 47.6 |
| 8 | 10 | 140 | 6.7 | 98.8 | 24.6 |

5.8. Test Example 2

Each metal hyaluronate (No. 12 to 15, No.11 corresponds to zinc hyaluronate of Example 1) was manufactured by the similar method to Example 1 except the metallic compounds and the mediums shown in Table 2 were used in place of the zinc acetate and the ethanol in Example 1. Tests No. 12 and 13 are reference examples.

[Table 2]

| No. | Metallic Compound | Medium | Yield of Metal Hyaluronate (%) | Metal Content per 1 g of Metal Hyaluronate (mg) |
|---|---|---|---|---|
| 11 | zinc acetate | ethanol | 108 | 72.2 |
| 12 | zinc chloride | ethanol | 68.0 | 12.3 |
| 13 | zinc sulfate | ethanol | 75.6 | 44.3 |
| 14 | zinc acetate | acetone | 104 | 71.7 |
| 15 | zinc acetate | methanol | 94.9 | 70.0 |

5.9. Test Example 3 (reference example)

Metal hyaluronate (No. 16 to 20) was manufactured by the similar method to Example 1 except the metallic compounds shown in Table 3 were used in place of the zinc acetate in Example 1. Furthermore, the each metal content (except zinc) per 1 g of the metal hyaluronate was measured by an ICP emission analyzing device. The each metal content (theoretical value) per 1 g of the metal hyaluronate shown in Table 3 was also calculated in accordance with the same method as Example 1. Additionally, the each value in the right-side brackets of the metal content (theoretical value) per 1 g of the metal hyaluronate was a ratio of a metal content (measurement value) per 1 g of the metal hyaluronate to a metal content (theoretical value) per 1 g of the metal hyaluronate, which is shown as a substitution rate (%).

[Table 3]

| No. | Metallic Compound | Yield of Metal Hyaluronate (%) | Metal Content per 1 g of Metal Hyaluronate (measured value) (mg) | Metal Content per 1 g of Metal Hyaluronate (theoretical value) (mg) |
|---|---|---|---|---|
| 16 | magnesium acetate | 102 | 24.7 | 31 (80 %) |
| 17 | copper acetate | 112 | 71.8 | 77 (93%) |
| 18 | cobalt acetate | 110 | 57.8 | 72 (80%) |
| 19 | silver acetate | 106 | 167 | 22 (75%) |
| 20 | calcium carbonate | 88 | 37.8 | 50 (76%) |

5.10. Example 3 (reference example)

Zinc hyaluronate (yield: 110 %, zinc content per 1 g of zinc hyaluronate: 61 mg, kinetic viscosity of 1 % aqueous solution:96 mm$^2$/s) was manufactured by the similar method to Example 1 except hyaluronic acid (molecular weight: approximately 1,200,000, average particle diameter: approximately 450 micrometers, manufactured by Kewpie Corporation) was used as a source in Example 1.

5.11. Example 4 (reference example)

Metal hyaluronate was manufactured by the similar method to Example 1 except 0.1 N hydrochloric acid was used in place of the zinc acetate and the ethanol in Example 1. The pH of the aqueous medium was 1.7.
The yield of the zinc hyaluronate obtained in Example 4 was 112 %, the zinc content per 1 g of zinc hyaluronate was 62.3 mg. In addition, the kinetic viscosity of the 1 % aqueous solution of the obtained zinc hyaluronate was 0.9 mm$^2$/s.

5.12. Test Example 4

Each zinc hyaluronate (No. 21 and 23 to 26, No.22 corresponds to zinc hyaluronate of Example 1) was manufactured by the similar method to Example 1 except the each hyaluronic acid having the each average molecular weight shown in Table 4 was used as a source in Example 1. Test No. 26 is a reference example.

[Table 4]

| No. | Average Molecular Weight | Average Particle Diameter (micrometer) | Yield of Zinc Hyaluronate (%) | Zinc Content per 1 g of Zinc Hyaluronate (mg) | Kinetic Viscosity of 1 % Aqueous Solution (mm$^2$/s) |
|---|---|---|---|---|---|
| 21 | 3,000 | 230 | 108 | 74.3 | 1.2 |
| 22 | 8,000 | 230 | 108 | 72.2 | 1.2 |
| 23 | 20,000 | 230 | 110 | 69.8 | 1.5 |
| 24 | 50,000 | 230 | 107 | 65.2 | 1.9 |
| 25 | 100,000 | 230 | 103 | 63.5 | 4.5 |
| 26 | 200,000 | 230 | 99 | 62.4 | 10.2 |

5.13. Test Example 5

Each zinc hyaluronate (No. 31 to 38) was manufactured by the similar method to Example 1 except hyaluronic acid (molecular weight: approximately 8,000, average particle diameter: approximately 200 micrometers, manufactured by Kewpie Corporation) was used as a source in Example 1 and the water amount and the medium (ethanol) amount in the water-containing medium before adding the hyaluronic acid were changed to those shown in Table 5. Test No. 38 is a reference example.

[Table 5]

| No. | Water Content in Water-containing Medium (mL) | Ethanol Content in Water-containing Medium (mL) | Water Proportion in Water-containing Medium (%) | Yield of Zinc Hyaluronate (%) | Zinc Content per 1 g of Zinc Hyaluronate (mg) | Kinetic Viscosity of 1 % Aqueous Solution (mm$^2$/s) |
|---|---|---|---|---|---|---|
| 31 | 80 | 70 | 53.3 | 94 | 73.4 | 1.2 |
| 32 | 70 | 80 | 46.7 | 101 | 70.3 | 1.2 |
| 33 | 60 | 90 | 40 | 104 | 69.4 | 1.2 |
| 34 | 50 | 100 | 33.3 | 106 | 66.7 | 1.2 |
| 35 | 40 | 110 | 26.7 | 105 | 62.8 | 1.2 |
| 36 | 30 | 120 | 20 | 103 | 55.4 | 1.2 |
| 37 | 20 | 130 | 13.3 | 102 | 47.6 | 1.2 |
| 38 | 10 | 140 | 6.7 | 98.8 | 24.6 | 1.2 |

5.14. Test Example 6

Each zinc hyaluronate (No. 42 to 45, No. 41 corresponds to the zinc hyaluronate of Example 1) was manufactured by the similar method to Example 1 except the zinc compounds and the mediums in the water-containing medium, which are shown in Table 6, were used as the zinc compound and the mediums in the water-containing medium in Example 1. Tests No. 42 and 43 are reference examples.

[Table 6]

| No. | Zinc Compound | Medium | Yield of Zinc Hyaluronate (%) | Zinc Content per 1 g of Metal Hyaluronate (mg) | Kinetic Viscosity of 1 % Aqueous Solution (mm$^2$/s) |
|---|---|---|---|---|---|
| 41 | zinc acetate | ethanol | 108 | 72.2 | 1.2 |
| 42 | zinc chloride | ethanol | 68.0 | 12.3 | 1.2 |
| 43 | zinc sulfate | ethanol | 75.6 | 44.3 | 1.2 |
| 44 | zinc acetate | acetone | 104 | 71.7 | 1.2 |
| 45 | zinc acetate | methanol | 94.9 | 70.0 | 1.2 |

5.15. Test Example 7

Each zinc hyaluronate (No. 51, 52, and 54 to 57, No. 53 corresponds to the zinc hyaluronate of Example 1) was manufactured by the similar method to Example 1 except the used amount of the zinc acetate, and the temperature and time of the reaction of the hyaluronic acid with the zinc acetate were adjusted to those shown in Table 6.

[Table 7]

| No. | Amount Used of Zinc Acetate (g) | Reaction Temperature (°C) | Reaction Time (minute) | Yield of Zinc Hyaluronate (%) | Zinc Content per 1 g of Zinc Hyaluronate (mg) | Kinetic Viscosity of 1 % Aqueous Solution (mm$^2$/s) |
|---|---|---|---|---|---|---|
| 51 | 1.5 | 40 | 60 | 109 | 58.1 | 1.2 |
| 52 | 3 | 25 | 60 | 109 | 70.5 | 1.2 |
| 53 | 3 | 40 | 60 | 108 | 72.2 | 1.2 |
| 54 | 3 | 60 | 60 | 103 | 72.9 | 1.2 |
| 55 | 3 | 25 | 30 | 109 | 68.9 | 1.2 |
| 56 | 3 | 40 | 30 | 108 | 70.4 | 1.2 |
| 57 | 3 | 60 | 30 | 103 | 71.4 | 1.2 |

5.16. Test Example 8 (Evaluation of Improvement Effect of Skin Roughness)

Each composition of test No. A, B and C was prepared in accordance with the following formulation described in Table 8. The units of the values shown in Table 8 are "weight %".

[Table 8]

| | Test Number A | Test Number B | Test Number C |
|---|---|---|---|
| Zinc Hyaluronate obtained in Example 1 | 0.1 | - | - |
| Zinc Hyaluronate obtained in Example 3 | - | 0.1 | - |
| Source Hyaluronic Acid used in Example 1 | - | - | 0.1 |
| 1,3-buthyleneglycol | 0.5 | 0.5 | 0.5 |
| water | balance | balance | balance |

The skin barrier function in the inside forearm of each four subjects was destroyed by tape-stripping the inside forearm to form a human skin-roughness-model skin. 0.3 mL of the compositions of test No. A, B, and C, and purified water as a control were applied on the each human skin-roughness-model skin respectively, three times per day for six consecutive days. The each transepidermides water loss (TEWL) before tape-stripping, immediately after tape-stripping, and on the day seven was measured by using Tewameter TM300. The each forearm was washed prior to 30 minutes. The recovery rate (%) of the transepidermides water loss (TEWL) was calculated in accordance with the following formula.

```
recovery rate (%) = {(TEWL value immediately after tape-stripping)
- (TEWL value on day seven)} / {(TEWL value immediately after
tape-stripping) - (TEWL value before tape-stripping)} x 100
```

The result is shown in FIG. 1. As is clear from FIG. 1, the zinc hyaluronate obtained in Example 1 exhibited an outstanding recovery effect of the amount of trans-epidermal water transpiration.

Furthermore, redness of the skin was evaluated in accordance with the following criteria described in Table 9. The result is shown in Table 10. As shown in Table 10, the zinc hyaluronate obtained in Example 1 exhibited an improvement effect of the redness of the skin.

[Table 9]

|  | mark |
|---|---|
| no redness | 0 |
| moderate redness | 1 |
| light redness | 2 |
| clear redness | 3 |
| distinct redness | 4 |

[Table 10]

|  | Before Tape-stripping | Immediately On Tape-stripping | Day Seven |
|---|---|---|---|
| Test Number A | 0 | 4 | 0 |
| Test Number B | 0 | 4 | 1 |
| Test Number C | 0 | 4 | 2 |
| Control | 0 | 4 | 3 |

According to Examples 1 to 4 and Test Examples 1 to 8, since the method for producing the metal hyaluronate comprises dispersing the hyaluronic acid and/or salt thereof in a water-containing medium comprising a metallic compound comprising at least one selected from a metal of a group 2 element, a metal of a group 12 element, and a metal of a group 13 element, it is clear that the metal hyaluronate can be obtained in a simple method in a high yield.

In contrast, in Comparative Examples 1 and 2, the reactions with the metallic compound (zinc acetate) were performed under a condition such that the source hyaluronic acid was the same as Example 1 and the source hyaluronic acid was completely dissolved. According to the result described above, the zinc hyaluronate of Example 1 exhibits higher yield than the zinc hyaluronate of Comparative Examples 1 and 2.

As well as Comparative Examples 1 and 2, Comparative Example 3 is an example of the reaction with the metallic compound (zinc acetate) performed under a condition such that the source hyaluronic acid was completely dissolved. In Example 2 and Comparative Example 3, the source hyaluronic acid having larger average molecular weight than the source hyaluronic acid used in Example 1 was used. In comparison with Example 2 and Comparative Example 3, since the source hyaluronic acid was dispersed in the water-containing medium comprising the metallic compound (zinc acetate) in Example 2, it is clear that the yield can be increased compared with the case that a reaction with metallic compound (zinc acetate) is performed under a condition such that the source hyaluronic acid is completely dissolved, even if the zinc hyaluronate is obtained by using the source hyaluronic acid having a large average molecular weight.

Additionally, in comparison with Example 2, the yield of the obtained zinc hyaluronate is higher and the zinc content is higher in Example 1. It is considered that since the source hyaluronic acid used in Example 1 has smaller average molecular weight than the hyaluronic acid used in Example 2, zinc ions penetrate into the molecular structure of the source hyaluronic acid more smoothly, and thus a complex can easily form by coordinating a carboxyl ion of the hyaluronic acid and a zinc ion, and as a result, the yield of the zinc hyaluronate can be increased and thus the zinc content can be increased.

It has also been found when the reaction was performed in the reaction liquid having a pH in a range of 3 to 10 (Example 1), the decrease in a kinetic viscosity (i.e., the decrease in molecular weight) is small and the yield of the zinc hyaluronate is higher compared with the case when the reaction was performed in the reaction liquid having a pH of less than 3 (Example 4).

5.17. Test Example 9

In this test example, a cosmetic preparation containing the zinc hyaluronate obtained in Example 1 was prepared according to the following formulation.

| | |
|---|---|
| zinc hyaluronate (Example 1) | 0.2% |
| sodium hyaluronate | 0.1% |
| hydrolyzed hyaluronic acid | 0.1% |
| collagen peptide | 0.1% |
| 1,3-butyleneglycol | 5.0% |
| glycerin | 3.0% |
| isostearyl alcohol | 0.1% |
| tocopherol acetate | 0.1% |
| POE (20) sorbitan monolaurylate | 0.5% |
| POE(15) lauryl alcohol ether | 0.5% |
| pyrrolidone zinc carboxylate | 0.1% |
| ethylparaben | 0.1% |
| methylparaben | 0.15% |
| ethanol | 5.0% |
| essence | proper quantity |
| purified water | balance |

5.18. Test Example 10 (reference example)

In this test example, milky lotion containing the zinc hyaluronate obtained in Example 2 was prepared according to the following formulation.

| | |
|---|---|
| zinc hyaluronate (Example 2) | 0.3% |
| pentylene glycol | 5.0% |
| glycerin | 3.0% |
| scualane | 5.0% |
| stearic acid | 0.5% |
| stearyl alcohol | 2.0% |
| vaseline | 4.0% |
| sorbitan stearate | 1.0% |
| POE(10) monostearate | 1.0% |
| carboxylvinyl polymer | 0.5% |
| poly cotanium-51 | 0.1% |
| methylparaben | 0.15% |
| propylparaben | 0.1% |
| potassium hydroxide | 0.1% |
| BHT | 0.02% |
| sodium EDTA-2 | 0.02% |
| essence | proper quantity |
| purified water | balance |

5.19. Test Example 11

In this test example, beauty essence pack (pasty peel-off type) containing the zinc hyaluronate obtained in Example 1 was prepared according to the following formulation.

| | |
|---|---|
| zinc hyaluronate (Example 1) | 0.5% |
| polyvinyl acetate emulsion | 17.0% |
| polyvinyl alcohol | 11.0% |
| sorbitol | 5.0% |
| polyethylene glycol 400 | 5.0% |
| scualane | 2.5% |
| POE sorbitan monostearate | 1.0% |
| titanium oxide | 4.0% |
| talc | 8.0% |
| ethanol | 8.0% |
| methylparaben | 0.15% |
| essence | proper quantity |
| purified water | balance |

5.20. Test Example 12

In this test example, facial wash (cleansing foam) containing the zinc hyaluronate obtained in Example 1 was prepared according to the following formulation.

| | |
|---|---|
| zinc hyaluronate (Example 1) | 0.2 % |
| cationized hyaluronic acid | 0.1 % |
| (manufactured by Kewpie corporation, brand name "Hyaloveil") | |
| glycerin | 10.0 % |
| polyethylene glycol 400 | 15.0 % |
| dipropylene glycol | 10.0 % |
| sodium lauroyl glutamate | 20.0 % |
| POE(2) monostearate | 5.0 % |
| palm fatty acid sodium glutamate | 8.0 % |
| alkyl betaine | 2.0 % |
| sodium EDTA-2 | 0.02 % |
| propylparaben | 0.1 % |
| methylparaben | 0.15 % |
| essence | proper quantity |
| purified water | balance |

5.21. Test Example 13

In this test example, sunscreen preparation (milky lotion) containing the zinc hyaluronate obtained in Example 1 was prepared according to the following formulation.

| | |
|---|---|
| zinc hyaluronate (Example 1) | 0.2 % |
| 1,3-butyleneglycol | 3.0 % |
| dipropylene glycol | 3.0 % |
| cyclomethicone | 5.0 % |
| dimethicone | 5.0 % |
| cetanol | 1.0 % |
| vaseline | 1.0 % |
| octyl methoxycinnamate | 5.0 % |
| titanium oxide | 2.0 % |
| zinc oxide | 2.0 % |
| sorbitan stearate | 1.0 % |

(continued)

| | |
|---|---|
| POE(20) sorbitan monostearate | 1.0 % |
| Phenoxyethanol | 0.8 % |
| Methylparaben | 0.1 % |
| Essence | proper quantity |
| purified water | balance |

5.22. Test Example 14

In this test example, lip balm containing the zinc hyaluronate obtained in Example 1 was prepared according to the following formulation.

| | |
|---|---|
| zinc hyaluronate (Example 1) | 0.1 % |
| microcrystalline wax | 1.5 % |
| ceresin | 12.0 % |
| scualane | 10.0 % |
| decamethyltetrasiloxane | 10.0 % |
| diisostearyl maleate | 5.0 % |
| candelilla wax | 2.0 % |
| vaseline | 8.0 % |
| glyceryl hydroxystearate | 2.0 % |
| menthol | 0.05 % |
| liquid paraffin | 1.0 % |
| tocopherol acetate | 0.1 % |
| tocopherol | 0.05 % |
| propylparaben | 0.1 % |
| essence | proper quantity |
| purified water | balance |

5.23. Test Example 15

In this test example, shampoo containing the zinc hyaluronate obtained in Example 1 was prepared according to the following formulation.

| | |
|---|---|
| zinc hyaluronate (Example 1) | 0.2 % |
| cationized hyaluronic acid | 0.1 % |
| (manufactured by Kewpie corporation, brand name "Hyaloveil") | |
| POE(20) sodium laureth sulfate | 11.0 % |
| sodium lauroyl aspartate | 10.0 % |
| palm oil fatty acid amide propyl betaine | 4.0 % |
| palm oil fatty acid mono ethanol amide | 2.0 % |
| sodium EDTA-2 | 0.1 % |
| sodium benzoate | 0.2 % |
| phenoxyethanol | 0.8 % |
| methylparaben | 0.1 % |
| essence | proper quantity |
| purified water | balance |

The embodiment of the invention has been explained as set out above.

**Claims**

1. A method for producing a zinc hyaluronate comprising dispersing a hyaluronic acid and/or salt thereof having an average molecular weight of 2,000 to 100,000 in a water-containing medium comprising zinc acetate at 15 to 60 °C at a pH of 3 to 9 for 10 minutes to 6 hours, wherein the average molecular weight is determined according to the method described in the description,

   wherein the amount of the source hyaluronic acid and/or salt thereof in the water-containing medium is 5 to 500 mg/ml,
   wherein the amount of the zinc acetate per volume of the water-containing medium is 5 to 500 mg/ml,
   wherein the used amount of the zinc acetate for one part of the hyaluronic acid and/or salt thereof is 0.05 to 20 parts,
   wherein an average particle diameter, as measured by laser diffraction measurement, of the hyaluronic acid and/or salt thereof to be dispersed in the water-containing medium is 50 to 500 micrometers,
   wherein a water content in the water-containing medium is 20 to 55 vol %, and
   wherein a medium constituting the water-containing medium is at least one selected from ethanol, methanol and acetone.

2. The method for producing the zinc hyaluronate according to claim 1, further comprising heating and drying a residue obtained by removing the water-containing medium.

3. The method for producing the zinc hyaluronate according to claim 1 or 2,
   wherein the dispersing is performed at 20 to 45 °C.

4. A method for producing a cosmetic preparation comprising a zinc hyaluronate, comprising adding the zinc hyaluronate obtainable by the method for producing the zinc hyaluronate according to any one of claims 1 to 3.

5. A zinc hyaluronate obtainable by the method according to any one of claims 1 to 3 with a zinc content of 50 to 130 mg per 1 g of the zinc hyaluronate, as measured by chelate titration according to the method described in the description, and a kinetic viscosity of a 1 % aqueous solution of the zinc hyaluronate is 0.3 to 5 $mm^2/s$, wherein the kinetic viscosity is calculated by:

   measuring a falling time of the aqueous solution in an incubator at 30 °C by using an Ubbelohde viscometer having such a coefficient that the falling time is 200 to 1,000 seconds; and
   multiplying the falling time by the coefficient.

6. A cosmetic preparation comprising the zinc hyaluronate according to claim 5.

7. The method for producing the zinc hyaluronate according to claim 1, wherein the water content in the water-containing medium is 20 to 40 vol %.

**Patentansprüche**

1. Verfahren zur Herstellung eines Zinkhyaluronats, umfassend Dispergieren einer Hyaluronsäure und/oder eines Salzes davon mit einem mittleren Molekulargewicht von 2.000 bis 100.000 in einem Zinkacetat umfassenden wasserhaltigen Medium für 10 Minuten bis 6 Stunden bei 15 bis 60 °C bei einem pH von 3 bis 9, wobei das mittlere Molekulargewicht nach dem in der Beschreibung beschriebenen Verfahren bestimmt wird,
   wobei die Menge der Ausgangshyaluronsäure und/oder des Salzes davon in dem wasserhaltigen Medium 5 bis 500 mg/ml beträgt,
   wobei die Menge des Zinkacetats pro Volumen des wasserhaltigen Mediums 5 bis 500 mg/ml beträgt,
   wobei die eingesetzte Menge des Zinkacetats pro einem Teil der Hyaluronsäure und/oder des Salzes davon 0,05 bis 20 Teile beträgt,
   wobei der mittlere Teilchendurchmesser, gemessen durch Laserbeugung, der in dem wasserhaltigen Medium zu dispergierenden Hyaluronsäure und/oder des Salzes davon 50 bis 500 Mikrometer beträgt,
   wobei der Wassergehalt in dem wasserhaltigen Medium 20 bis 55 Vol.-% beträgt, und
   wobei ein das wasserhaltige Medium bildendes Medium wenigstens eines ist, das ausgewählt ist aus Ethanol, Methanol und Aceton.

**2.** Verfahren zur Herstellung des Zinkhyaluronats nach Anspruch 1, weiterhin umfassend Erhitzen und Trocknen eines nach Entfernen des wasserhaltigen Mediums erhaltenen Rückstands.

**3.** Verfahren zur Herstellung des Zinkhyaluronats nach Anspruch 1 oder 2, wobei das Dispergieren bei 20 bis 45 °C durchgeführt wird.

**4.** Verfahren zur Herstellung eines ein Zinkhyaluronat umfassenden Kosmetikpräparats, umfassend Zugeben des durch das Verfahren zur Herstellung des Zinkhyaluronats nach einem der Ansprüche 1 bis 3 erhältlichen Zinkhyaluronats.

**5.** Zinkhyaluronat, erhältlich durch das Verfahren nach einem der Ansprüche 1 bis 3 mit einem Zinkgehalt von 50 bis 130 mg pro 1 g des Zinkhyaluronats, gemessen durch Chelattitration nach dem in der Beschreibung beschriebenen Verfahren, wobei die kinetische Viskosität einer 1%igen wässrigen Lösung des Zinkhyaluronats 0,3 bis 5 mm$^2$/s beträgt, wobei die kinetische Viskosität berechnet wird durch:

Messen der Fallzeit der wässrigen Lösung in einem Inkubator bei 30 °C mittels eines Ubbelohde-Viskosimeters mit einem solchen Koeffizienten, dass die Fallzeit 200 bis 1.000 Sekunden beträgt; und Multiplizieren der Fallzeit mit dem Koeffizienten.

**6.** Kosmetikpräparat, umfassend das Zinkhyaluronat nach Anspruch 5.

**7.** Verfahren zur Herstellung des Zinkhyaluronats nach Anspruch 1, wobei der Wassergehalt in dem wasserhaltigen Medium 20 bis 40 Vol.-% beträgt.

## Revendications

**1.** Procédé de production d'un hyaluronate de zinc comprenant la dispersion d'un acide hyaluronique et/ou d'un sel de celui-ci ayant un poids moléculaire moyen de 2 000 à 100 000, dans un milieu contenant de l'eau et comprenant de l'acétate de zinc à 15 à 60 °C à un pH de 3 à 9 pendant 10 minutes à 6 heures, dans lequel le poids moléculaire moyen est déterminé selon le procédé décrit dans la description, dans lequel la quantité de l'acide hyaluronique source et/ou d'un sel de celui-ci dans le milieu contenant de l'eau est de 5 à 500 mg/ml, dans lequel la quantité de l'acétate de zinc par volume du milieu contenant de l'eau est de 5 à 500 mg/ml, dans lequel la quantité utilisée de l'acétate de zinc pour une partie de l'acide hyaluronique et/ou d'un sel de celui-ci est de 0,05 à 20 parties, dans lequel le diamètre particulaire moyen, comme mesuré par mesure par diffraction au laser, de l'acide hyaluronique et/ou d'un sel de celui-ci à disperser dans le milieu contenant de l'eau est de 50 à 500 micromètres, dans lequel la teneur en eau dans le milieu contenant de l'eau est de 20 à 55 % en volume et dans lequel un milieu constituant le milieu contenant de l'eau est au moins l'un choisi parmi l'éthanol, le méthanol et l'acétone.

**2.** Procédé de production du hyaluronate de zinc selon la revendication 1, comprenant en outre le chauffage et le séchage d'un résidu obtenu par élimination du milieu contenant de l'eau.

**3.** Procédé de production du hyaluronate de zinc selon la revendication 1 ou 2, dans lequel la dispersion est effectuée à une température de 20 à 45 °C.

**4.** Procédé de production d'une préparation cosmétique comprenant un hyaluronate de zinc, comprenant l'addition du hyaluronate de zinc qui peut être obtenu par le procédé de production du hyaluronate de zinc selon l'une quelconque des revendications 1 à 3.

**5.** Hyaluronate de zinc qui peut être obtenu par le procédé selon l'une quelconque des revendications 1 à 3 avec une teneur en zinc de 50 à 130 mg pour 1 g du hyaluronate de zinc, comme mesuré par titrage du chélate selon le procédé dans la description, et une viscosité cinétique d'une solution aqueuse à 1 % du hyaluronate de zinc est de 0,3 à 5 mm$^2$/s, dans lequel la viscosité cinétique est calculée :

en mesurant un temps de chute de la solution aqueuse dans une incubateur à 30 °C en utilisant un viscosimètre d'Ubbelohde ayant un coefficient tel que le temps de chute soit de 200 à 1 000 secondes ; et
en multipliant le temps de chute par le coefficient.

6. Préparation cosmétique comprenant le hyaluronate de zinc selon la revendication 5.

7. Procédé de production du hyaluronate de zinc selon la revendication 1, dans lequel la teneur en eau du milieu contenant de l'eau est de 20 à 40 % en volume.

# FIGURE 1

**REFERENCES CITED IN THE DESCRIPTION**

**Patent documents cited in the description**

- JP 2571312 B
- CN 1760214 A

- JP 2009278259 A